# EUROPEAN PATENT SPECIFICATION

(11) **EP 3 747 360 B1**
(45) Date of publication and mention of the grant of the patent: **19.07.2023**
(21) Application number: 20178450.1
(22) Date of filing: 05.06.2020
(51) Int. Cl.: A61B 5/11, A61B 5/103, A61B 5/00, G06T 7/00, G06V 40/20, G09B 19/00, G16H 20/30

(54) **WALKING TRAINING SYSTEM, NON-TRANSITORY STORAGE MEDIUM STORING CONTROL PROGRAM FOR WALKING TRAINING SYSTEM AND CONTROL METHOD FOR WALKING TRAINING SYSTEM**
GEHTRAININGSSYSTEM, NICHTTRANSITORISCHES SPEICHERMEDIUM MIT EINEM STEUERUNGSPROGRAMM FÜR EIN GEHTRAININGSSYSTEM UND STEUERUNGSVERFAHREN FÜR EIN GEHTRAININGSSYSTEM
SYSTÈME D'ENTRAÎNEMENT À LA MARCHE, SUPPORT D'INFORMATIONS NON TRANSITOIRE STOCKANT UN PROGRAMME DE COMMANDE POUR LE SYSTÈME D'ENTRAÎNEMENT À LA MARCHE ET PROCÉDÉ DE COMMANDE POUR LE SYSTÈME D'ENTRAÎNEMENT À LA MARCHE

(30) Priority: 07.06.2019 JP 2019106943
(43) Date of publication of application: 09.12.2020
(73) Proprietor: TOYOTA JIDOSHA KABUSHIKI KAISHA, TOYOTA-SHI, AICHI-KEN 471-8571 (JP)
(72) Inventor: NAKASHIMA, Issei, Aichi-ken, 471-8571 (JP); NAKAMURA, Takuma, Aichi-ken, 471-8571 (JP); SAITOH, Eiichi, Toyoake, Aichi 470-1192 (JP); SAITO, Akihiro, Toyoake, Aichi 470-1192 (JP); HIRANO, Satoshi, Toyoake, Aichi 470-1192 (JP); TANABE, Shigeo, Toyoake, Aichi 470-1192 (JP); II, Takuma, Toyoake, Aichi 470-1192 (JP)
(74) Representative: D Young & Co LLP

(56) References cited:
- EP-A1- 3 489 965
- WO-A1-2018/131045
- JP-A- 2015 104 397
- US-A1- 2007 275 830
- US-A1- 2018 325 467

## Description

### BACKGROUND OF THE INVENTION

### 1. Field of the Invention

The invention relates to a walking training system, a non-transitory storage medium storing a control program for the walking training system, and a control method for the walking training system.

### 2. Description of Related Art

There is known an apparatus configured to image scenes showing a trainee who undergoes walking training, generate image data corresponding to a motion of a skeleton, and make determination on a walking action of the trainee (see, for example, Japanese Unexamined Patent Application Publication No. 2015-104397 (JP 2015-104397 A)).

US 2018/325467 A1 relates to a `Gait motion display system and program'. US 2007/275830 A1 relates to a `Gait training system using motion analysis'. JP 2015 104397 A relates to a `Gait training apparatus and a gait training system'.

### SUMMARY OF THE INVENTION

The trainee who undergoes the walking training may want to quickly and intuitively review what is inappropriate in comparison with a normal gait after the training is finished. If the trainee reviews all training scenes in a video, a checking time equal to the training time is required. Even if a fast-forward function is used, a long time is required for a search unless the trainee grasps the point where a video to be reviewed is present.

The invention provides a walking training system and the like in which a trainee can quickly and intuitively review training after the training is finished.

A first aspect of the invention relates to a walking training system. The walking training system includes a treadmill, a camera system, a determination unit, an image processing unit, and a display control unit. The treadmill is configured to prompt a trainee to walk. The camera system is configured to image the trainee at an angle of view at which at least a part of a gait of the trainee walking on the treadmill is recognizable. The determination unit is configured to determine whether abnormal walking occurs in the trainee based on a plurality of predetermined determination criteria. The image processing unit is configured to clip, as a video clip, a video in a predetermined period including a timing when the determination unit determines that the abnormal walking occurs from a video captured by the camera system, and perform image processing to emphasize an abnormal part of the abnormal walking determined by the determination unit. The display control unit is configured to cause a display unit to display the video clip subjected to the image processing.

The displayed video clip includes a scene at the timing of the determination that the abnormal walking occurs. Moreover, the abnormal part that is the cause of the determination is emphasized. Therefore, the trainee can quickly and intuitively review what is inappropriate.

In the walking training system of the aspect described above, the camera system may include a plurality of cameras configured to image the trainee in different directions. The image processing unit may be configured to determine which of videos captured by the cameras is clipped as the video clip based on a determination criterion determined to be applicable by the determination unit out of the determination criteria. The direction in which the abnormality is clearly observed differs depending on how the walking is abnormal. By determining which of the videos captured by the cameras is used for creating the video clip in association with the determination criterion, the trainee can recognize the abnormal walking more clearly.

In the walking training system of the aspect described above, the image processing unit may be configured such that, when the determination criterion determined to be applicable by the determination unit is a specific determination criterion, videos captured by the cameras are clipped as the video clips. The display control unit may be configured to cause the display unit to display the video clips in synchronization with each other. Depending on the type of the abnormal walking, the scene of the abnormal walking may be well understandable, for example, when a scene from the front and a scene from the side are observed simultaneously. When the videos are displayed in synchronization with each other, the trainee can recognize the scene of his/her abnormal walking more accurately.

In the walking training system of the aspect described above, the image processing unit may be configured to clip, as a comparative video, a video in a normal-walking period in which the determination unit does not determine that the abnormal walking occurs. The display control unit may be configured to cause the display unit to display the comparative video such that the comparative video adjoins the video clip. When the abnormal-walking video and the normal-walking video are displayed adj acent to each other, the trainee can recognize the degree of the abnormal walking more accurately.

In the walking training system of the aspect described above, the image processing unit may be configured to determine a clipping period for the video clip based on the determination criterion determined to be applicable by the determination unit. The time during which the abnormal scene continues differs depending on the type of the abnormal walking. Therefore, it is appropriate that the clipping period be determined based on the determination criterion in order that the trainee can recognize the entire scene of the abnormal walking.

In the walking training system of the aspect described above, the image processing unit may be configured to determine the clipping period for the video clip based on a walking cycle of the trainee. Since the leg motion speed differs among trainees, the trainee can recognize the entire scene of the abnormal walking more securely when the video clip is generated based on the walking cycle of the trainee.

A second aspect of the invention relates to a non-transitory storage medium storing a control program for a walking training system. The walking training system includes a treadmill and a camera system. The treadmill is configured to prompt a trainee to walk. The camera system is configured to image the trainee at an angle of view at which at least a part of a gait of the trainee walking on the treadmill is recognizable. The non-transitory storage medium stores instructions that are executable by one or more processors and that cause the one or more processors to perform the following functions. The functions include determining whether abnormal walking occurs in the trainee based on a plurality of predetermined determination criteria, clipping, as a video clip, a video in a predetermined period including a timing when determination is made that the abnormal walking occurs from a video captured by the camera system, performing image processing on the video clip to emphasize an abnormal part of the determined abnormal walking, and causing a display unit to display the video clip subjected to the image processing. With the walking training system in which the control program is executed, the trainee can quickly and intuitively review what is inappropriate.

A third aspect of the invention relates to a control method for a walking training system. The walking training system includes a treadmill and a camera system. The treadmill is configured to prompt a trainee to walk. The camera system is configured to image the trainee at an angle of view at which at least a part of a gait of the trainee walking on the treadmill is recognizable. The control method includes determining whether abnormal walking occurs in the trainee based on a plurality of predetermined determination criteria, clipping, as a video clip, a video in a predetermined period including a timing when determination is made that the abnormal walking occurs from a video captured by the camera system, performing image processing on the video clip to emphasize an abnormal part of the determined abnormal walking, and causing a display unit to display the video clip subjected to the image processing.

According to the invention, it is possible to provide the walking training system and the like in which the trainee can quickly and intuitively review the training after the training is finished.

### BRIEF DESCRIPTION OF THE DRAWINGS

Features, advantages, and technical and industrial significance of exemplary embodiments of the invention will be described below with reference to the accompanying drawings, in which like signs denote like elements, and wherein:
FIG. 1 is a schematic perspective view of a walking training apparatus according to an embodiment;
FIG. 2 is a schematic perspective view of a walking assistance device;
FIG. 3 is a diagram illustrating the system configuration of the walking training apparatus;
FIG. 4 is an illustration of a display example of an exercise screen on a training monitor during execution of training;
FIG. 5 is an illustration of a display example of a replay screen on the training monitor after the execution of the training;
FIG. 6 is an explanatory drawing of a reproducing period of an abnormal-walking video clip;
FIG. 7 is an explanatory drawing of a reproducing period of a normal-walking video clip;
FIG. 8 is an illustration of a display example of another replay screen on the training monitor after the execution of the training;
FIG. 9 is a diagram illustrating a creation criterion table to be used for creating video clips; and
FIG. 10 is a diagram illustrating a processing flow in a series of operations in the execution of the training.

### DETAILED DESCRIPTION OF EMBODIMENTS

An embodiment of the invention is described below. The invention according to the claims is not limited to the embodiment described below. All constituent elements described in the embodiment are not essential to solve the problem.

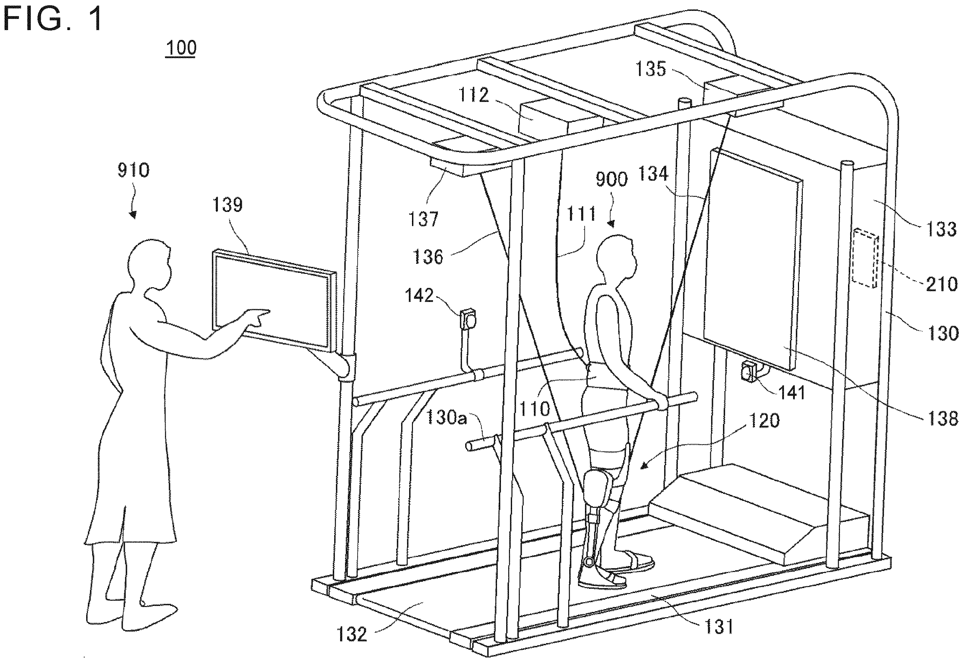

FIG. 1 is a schematic perspective view of a walking training apparatus 100 according to this embodiment. The walking training apparatus 100 is an example of a walking training system. A trainee 900 undergoes walking training by using the walking training apparatus 100. The trainee 900 is a hemiplegia patient suffering from paralysis in one leg. The walking training apparatus 100 mainly includes a control panel 133, a treadmill 131, and a walking assistance device 120. The control panel 133 is attached to a frame 130 that constitutes the entire skeleton. The trainee 900 walks on the treadmill 131. The walking assistance device 120 is attached to an affected leg of the trainee 900, which is a paralytic leg.

The frame 130 is provided upright on the treadmill 131 installed on a floor. The treadmill 131 rotates a ring-shaped belt 132 by using a motor (not illustrated). The treadmill 131 prompts the trainee 900 to walk. The trainee 900 who undergoes walking training stands on the belt 132, and attempts to walk in synchronization with movement of the belt 132.

The frame 130 supports the control panel 133, a training monitor 138, and the like. The control panel 133 houses an overall control unit 210 configured to control motors and sensors. For example, the training monitor 138 is a liquid crystal display panel configured to display information related to execution of training for the trainee 900. The training monitor 138 is installed so that the trainee 900 can view the training monitor 138 while walking on the belt 132 of the treadmill 131. The frame 130 supports a front tension unit 135 near the front of an area over the head of the trainee 900, a harness tension unit 112 near the area over the head, and a rear tension unit 137 near the rear of the area over the head. The frame 130 includes handrails 130a to be gripped by the trainee 900.

A front camera unit 141 images the trainee 900 from the front at an angle of view at which a gait of the trainee 900 is recognizable. A side camera unit 142 images the trainee 900 from the side at an angle of view at which the gait of the trainee 900 is recognizable. Each of the front camera unit 141 and the side camera unit 142 of this embodiment includes a set of a lens and an imaging device to achieve an angle of view at which each of the front camera unit 141 and the side camera unit 142 can capture the entire body of the trainee 900 standing on the belt 132, including his/her head. For example, the imaging device is a complementary metal-oxide semiconductor (CMOS) image sensor, and converts an optical image formed on an imaging plane into an image signal. The front camera unit 141 is installed near the training monitor 138 to face the trainee 900. The side camera unit 142 is installed on the handrail 130a to capture the trainee 900 from the side.

A front wire 134 has one end coupled to a reeling mechanism of the front tension unit 135, and the other end coupled to the walking assistance device 120. The reeling mechanism of the front tension unit 135 reels or unreels the front wire 134 in response to a motion of the affected leg by turning ON or OFF a motor (not illustrated). Similarly, a rear wire 136 has one end coupled to a reeling mechanism of the rear tension unit 137, and the other end coupled to the walking assistance device 120. The reeling mechanism of the rear tension unit 137 reels or unreels the rear wire 136 in response to a motion of the affected leg by turning ON or OFF a motor (not illustrated). Through the cooperative operation of the front tension unit 135 and the rear tension unit 137, a load of the walking assistance device 120 is balanced so as not to burden the affected leg. Further, the swing of the affected leg is assisted depending on a set level.

An operator 910 who is a training assistant sets a high assistance level for a trainee suffering from severe paralysis. The operator 910 is a physical therapist or a doctor having authority to select, change, or add setting items of the walking training apparatus 100. When the high assistance level is set, the front tension unit 135 reels the front wire 134 with a relatively great force in synchronization with a timing to swing the affected leg. If the training proceeds and no assistance is needed, the operator sets a minimum assistance level. When the minimum assistance level is set, the front tension unit 135 reels the front wire 134 with a force for canceling the self-weight of the walking assistance device 120 in synchronization with a timing to swing the affected leg.

The walking training apparatus 100 includes a safety device having a safety attachment 110, a harness wire 111, and the harness tension unit 112 as main components. The safety attachment 110 is a belt to be attached around the waist of the trainee 900, and is fixed to the waist with, for example, a hook-and-loop fastener. The harness wire 111 has one end coupled to the safety attachment 110, and the other end coupled to a reeling mechanism of the harness tension unit 112. The reeling mechanism of the harness tension unit 112 reels or unreels the harness wire 111 by turning ON or OFF a motor (not illustrated). The safety device having this structure is configured such that, when the trainee 900 loses his/her balance significantly, the harness wire 111 is reeled and the safety attachment 110 supports the upper body of the trainee 900 in response to an instruction from the overall control unit 210 that detects the motion of the trainee 900.

A management monitor 139 is a display device attached to the frame 130 and used by the operator 910 for monitoring and operation. For example, the management monitor 139 is a liquid crystal display panel having a touch panel superimposed on its surface. The management monitor 139 displays various menu items related to training settings, various parameters during the training, and results of the training. The operator 910 selects, changes, or adds the setting items via the touch panel, a keyboard (not illustrated), or the like. The management monitor 139 is installed at a position where the trainee 900 cannot view the display from a training position on the treadmill 131. A support for the management monitor 139 may have a rotation mechanism configured to flip the display screen when the operator 910 intends to show the display screen for the trainee 900.

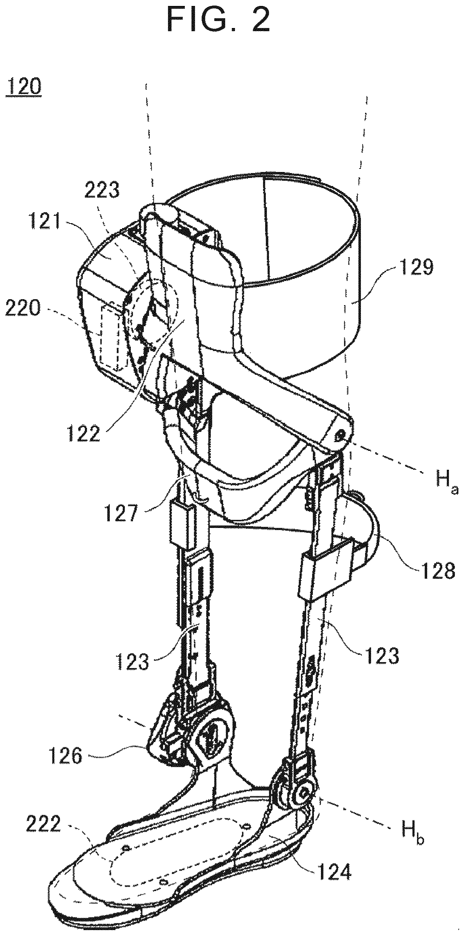

The walking assistance device 120 is attached to the affected leg of the trainee 900, and assists walking of the trainee 900 by reducing loads caused by stretching and bending a knee joint of the affected leg. FIG. 2 is a schematic perspective view of the walking assistance device 120. The walking assistance device 120 mainly includes a control unit 121, a plurality of frames, and a load sensor 222. The frames support respective parts of the affected leg. The load sensor 222 detects a load on a sole.

The control unit 121 includes an assistance control unit 220 and a motor (not illustrated). The assistance control unit 220 controls the walking assistance device 120. The motor generates a driving force for assisting stretching and bending motions of the knee joint. The frames that support respective parts of the affected leg include an upper thigh frame 122, a lower thigh frame 123, a foot frame 124, a front coupling frame 127, and a rear coupling frame 128. The lower thigh frame 123 is pivotably coupled to the upper thigh frame 122. The foot frame 124 is pivotably coupled to the lower thigh frame 123. The front wire 134 is coupled to the front coupling frame 127. The rear wire 136 is coupled to the rear coupling frame 128. The front coupling frame 127 extends in a lateral direction in front of an upper thigh, and both ends of the front coupling frame 127 are connected to the upper thigh frame 122. The rear coupling frame 128 extends in the lateral direction behind a lower thigh, and both ends of the rear coupling frame 128 are connected to the lower thigh frame 123 extending in a vertical direction.

The upper thigh frame 122 and the lower thigh frame 123 pivot relative to each other about an illustrated hinge axis Hₐ. The motor of the control unit 121 rotates in response to an instruction from the assistance control unit 220 to apply a force so that the upper thigh frame 122 and the lower thigh frame 123 are opened or closed relative to each other about the hinge axis Hₐ. An angle sensor 223 housed in the control unit 121 is, for example, a rotary encoder, and detects an angle between the upper thigh frame 122 and the lower thigh frame 123 about the hinge axis Hₐ. The lower thigh frame 123 and the foot frame 124 pivot relative to each other about an illustrated hinge axis H_{b}. An adjustment mechanism 126 preadjusts an angle range in which the lower thigh frame 123 and the foot frame 124 pivot relative to each other.

The upper thigh frame 122 includes an upper thigh belt 129. The upper thigh belt 129 is provided integrally with the upper thigh frame, and is attached around the upper thigh of the affected leg to fix the upper thigh frame 122 to the upper thigh. The upper thigh belt 129 restrains the entire walking assistance device 120 from being misaligned from the leg of the trainee 900.

The load sensor 222 is embedded in the foot frame 124. The load sensor 222 detects the magnitude and distribution of a vertical load on the sole of the trainee 900. For example, the load sensor 222 is a load detection sheet of a resistance variation detection type, in which electrodes are arranged in a matrix.

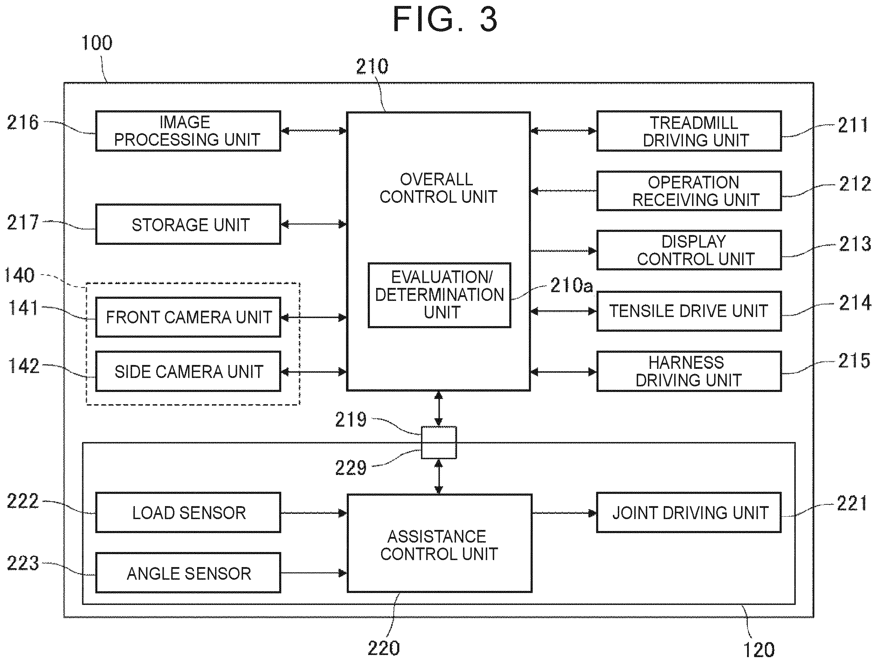

Next, the system configuration of the walking training apparatus 100 is described. FIG. 3 is a system configuration diagram of the walking training apparatus 100. For example, the overall control unit 210 is a microprocessor (MPU), and controls the entire apparatus by executing a control program read from a storage unit 217. A treadmill driving unit 211 includes a motor for rotating the belt 132, and a drive circuit for the motor. The overall control unit 210 controls the rotation of the belt 132 by transmitting a drive signal to the treadmill driving unit 211. For example, the rotation speed of the belt 132 is adjusted depending on a set training level.

An operation receiving unit 212 includes various input devices configured to receive an input operation from the trainee 900 or the operator 910 and transmit an operation signal to the overall control unit 210. The touch panel superimposed on the management monitor 139 is an example of the operation receiving unit 212. The trainee 900 or the operator 910 operates operation buttons or the touch panel of the operation receiving unit 212 or an attached remote control to give an instruction to turn ON or OFF the power or an instruction to start training, to input values related to settings, or to select a menu item. The operation receiving unit 212 may include a voice interaction device or an image recognition device. A display control unit 213 generates a display video and displays the display video on the training monitor 138 or the management monitor 139 in response to a control signal from the overall control unit 210. For example, the display control unit 213 generates a video showing progress of training. Details are described later.

A tensile drive unit 214 includes a motor for pulling the front wire 134, a drive circuit for the motor, a motor for pulling the rear wire 136, and a drive circuit for the motor. The overall control unit 210 controls reeling of the front wire 134 and reeling of the rear wire 136 by transmitting drive signals to the tensile drive unit 214. In addition to the control on the reeling operations, the overall control unit 210 controls tensile forces of the respective wires by controlling driving torques of the motors. For example, the overall control unit 210 determines a timing when the affected leg is switched from a stance phase to a swing phase based on a detection result from the load sensor 222, and increases or reduces the tensile forces of the respective wires in synchronization with the timing, thereby assisting the swing of the affected leg.

A harness driving unit 215 includes a motor for pulling the harness wire 111, and a drive circuit for the motor. The overall control unit 210 controls reeling of the harness wire 111 and a tensile force of the harness wire 111 by transmitting a drive signal to the harness driving unit 215. For example, when the trainee 900 loses his/her balance significantly, the overall control unit 210 reels the harness wire 111 by a predetermined amount to restrain the trainee from falling down.

An image processing unit 216 generates image data by performing image processing on image signals received from the front camera unit 141 and the side camera unit 142 in response to a control signal from the overall control unit 210. The image processing unit 216 also analyzes specific images by performing image processing on image signals received from the front camera unit 141 and the side camera unit 142 in response to an instruction from the overall control unit 210. For example, the positions of both shoulders and the position of a hip joint can be detected based on information on an extracted edge. The positional information serves as basic information for evaluating conditions of a body core, legs, feet, and the like during a leg motion of the trainee 900. The image processing unit 216 also generates a video clip by clipping a part of a video in the generated image data and performing image processing. Details are described later.

The storage unit 217 includes a non-volatile storage medium such as a solidstate drive. The non-volatile storage medium stores not only the control program for controlling the walking training apparatus 100, but also various parameters, functions, lookup tables, and a creation criterion table described later for use in control or calculation. The storage unit 217 further includes a volatile storage medium such as a dynamic random access memory (DRAM). The volatile storage medium functions not only as a working memory to be used when the overall control unit 210 or the like executes calculation and processing, but also as a temporary memory configured to temporarily store training evaluations and video data.

A camera system 140 provided in the walking training apparatus 100 includes the front camera unit 141 and the side camera unit 142 as a plurality of camera units configured to image the trainee in different directions. The front camera unit 141 and the side camera unit 142 repeat imaging operations and output image signals to the image processing unit 216 in response to a control signal from the overall control unit 210. The overall control unit 210 also serves as a function executing unit configured to execute various types of control and various types of calculation related to control. An evaluation/determination unit 210a evaluates rehabilitation training executed by the trainee 900, and makes determination on abnormal walking of the trainee 900 with reference to predetermined determination criteria. For example, the evaluation/determination unit 210a functions as a determination unit configured to determine whether a walking action is normal or abnormal by applying an analysis result from the image processing unit 216 to the determination criteria. Specific determination criteria and a specific determination method are described later.

As described above, the walking assistance device 120 is attached to the affected leg of the trainee 900. The walking training apparatus 100 includes a communication connection interface (IF) 219 connected to the overall control unit 210 to give instructions to the walking assistance device 120 and receive sensor information. The walking assistance device 120 is provided with a communication connection IF 229 connected to the communication connection IF 219 by wire or wireless. The communication connection IF 229 is connected to the assistance control unit 220 of the walking assistance device 120. Each of the communication connection IFs 219 and 229 is a communication interface such as a wireless local area network (LAN) conforming to a communication standard.

For example, the assistance control unit 220 is an MPU, and controls the walking assistance device 120 by executing a control program provided from the overall control unit 210. The assistance control unit 220 notifies the overall control unit 210 of the condition of the walking assistance device 120 via the communication connection IFs 219 and 229. The assistance control unit 220 starts or stops the walking assistance device 120 in response to an instruction from the overall control unit 210.

A joint driving unit 221 includes the motor of the control unit 121 and a drive circuit for the motor. The assistance control unit 220 transmits a drive signal to the joint driving unit 221 to apply a force so that the upper thigh frame 122 and the lower thigh frame 123 are opened or closed relative to each other about the hinge axis Hₐ. This operation assists stretching and bending motions of the knee, and restrains unintended bending on the knee. As described above, the load sensor 222 detects the magnitude and distribution of a vertical load on the sole of the trainee 900, and transmits a detection signal to the assistance control unit 220.

The assistance control unit 220 receives and analyzes the detection signal to determine whether the affected leg is in a swing phase or a stance phase or to estimate switching between the swing phase and the stance phase. As described above, the angle sensor 223 detects an angle between the upper thigh frame 122 and the lower thigh frame 123 about the hinge axis Hₐ, and transmits a detection signal to the assistance control unit 220. The assistance control unit 220 receives the detection signal, and calculates an open angle of the knee joint.

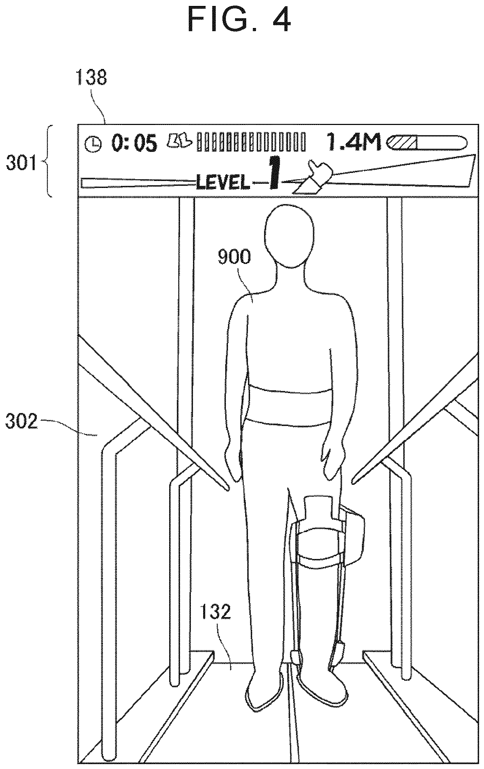

Next, the display on the training monitor 138 is described. FIG. 4 illustrates a display example of an exercise screen on the training monitor 138 during execution of training. A status field 301 is provided at an uppermost part of the training monitor 138. Status information in the execution of the training is displayed in the status field 301. The status information includes a duration, a walking distance, a training level, and various indicators. The duration is a time from the start of execution, and is measured by using a timer (not illustrated). The walking distance is measured based on a cumulative rotation amount of the belt 132 rotated by the treadmill driving unit 211. The training level indicates difficulty in the execution of the training, and is updated every time the trainee satisfies a preset criterion. The difficulty in the execution of the training is determined based on the rotation speed of the belt 132 and the assistance amount of the walking assistance device 120.

A camera image 302 is displayed in a field other than the status field 301 in the training monitor 138. The camera image 302 is an image of the entire trainee 900 photographed by the front camera unit 141 or the side camera unit 142, and is displayed as a real-time video of, for example, 60 fps. The trainee 900 can view himself/herself as the real-time video during the execution of the training. When the image captured by the front camera unit 141 is displayed, the camera image 302 is preferably a mirror image as in the illustration from the viewpoint of visibility because the trainee 900 faces the training monitor 138.

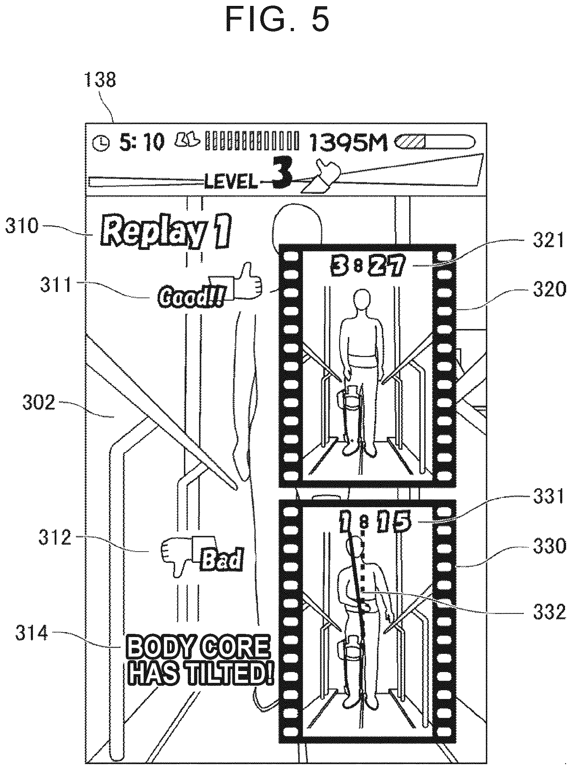

FIG. 5 illustrates a display example of a replay screen on the training monitor 138 after the execution of the training. The overall control unit 210 displays a video clip on the training monitor 138 after the execution of the training. The video clip is generated by clipping a moving image captured during the execution of the training.

In the replay screen, a title 310, a video clip 320 when the gait is appropriate, and a video clip 330 including a time when an abnormality is detected are superimposed on the continuously displayed camera image 302. For example, the title 310 reads "Replay 1" to indicate a reproduced screen.

The video clip 330 is generated from a video captured by the camera system 140 so as to include a time when the evaluation/determination unit 210a determines that any predetermined abnormal-walking determination criterion is satisfied. An emphasis 332 is superimposed on the clipped video as a computer graphics (CG) image to emphasize an abnormal part determined by the evaluation/determination unit 210a. The video clip 330 is obtained when the evaluation/determination unit 210a determines that a body core tilt occurs in the trainee 900. Therefore, CG images of a dashed reference line and a wide body core line are superimposed on the upper body of the trainee as the emphasis 332. The video clip is provided in order that the trainee 900 may review some scenes in the training after the execution of the training. Therefore, the video clip is not displayed as a mirror image like the video during the execution of the training in FIG. 4.

A time indication 331 is superimposed on the video clip 330 to indicate a time when the frame images are captured. An attribute icon 312 and a determination comment 314 of the video clip 330 are displayed near the video clip 330. The attribute icon 312 indicates an attribute of the video clip 330. Since the video clip 330 shows a scene of the abnormal walking, a "Bad" icon is selected. The determination comment 314 is superimposed when the video clip 330 shows the scene of the abnormal walking. A comment associated with the determination criterion determined by the evaluation/determination unit 210a is selected as the determination comment 314. Since the evaluation/determination unit 210a determines that the body core tilt occurs in the trainee 900, a corresponding comment "Body core has tilted!" is selected.

The video clip 320 is obtained by clipping, from the video captured by the camera system 140, a video in a normal-walking period in which the evaluation/determination unit 210a does not determine that abnormal walking occurs. The video clip 320 is a comparative video to the video clip 330, and therefore preferably adjoins the video clip 330. In this case, the video clip 320 and the video clip 330 vertically adjoin each other. Since the trainee 900 may desirably recognize the scene of the abnormal walking, the video clip showing the abnormal walking may be displayed in a size larger than that of the video clip showing the normal walking.

A time indication 321 is superimposed on the video clip 320 to indicate a time when the frame images are captured. An attribute icon 311 of the video clip 320 is displayed near the video clip 320. The attribute icon 311 indicates an attribute of the video clip 320. Since the video clip 320 shows a scene of the normal walking, a "Good" icon is selected.

The video clip 320 and the video clip 330 are simultaneously reproduced so that walking cycles are synchronized to some extent. Since the video clip 320 and the video clip 330 are reproduced in this manner in the replay screen together with auxiliary information, the trainee 900 can accurately recognize what is inappropriate and the degree of inappropriateness in the previous execution of the training in comparison with the appropriate condition.

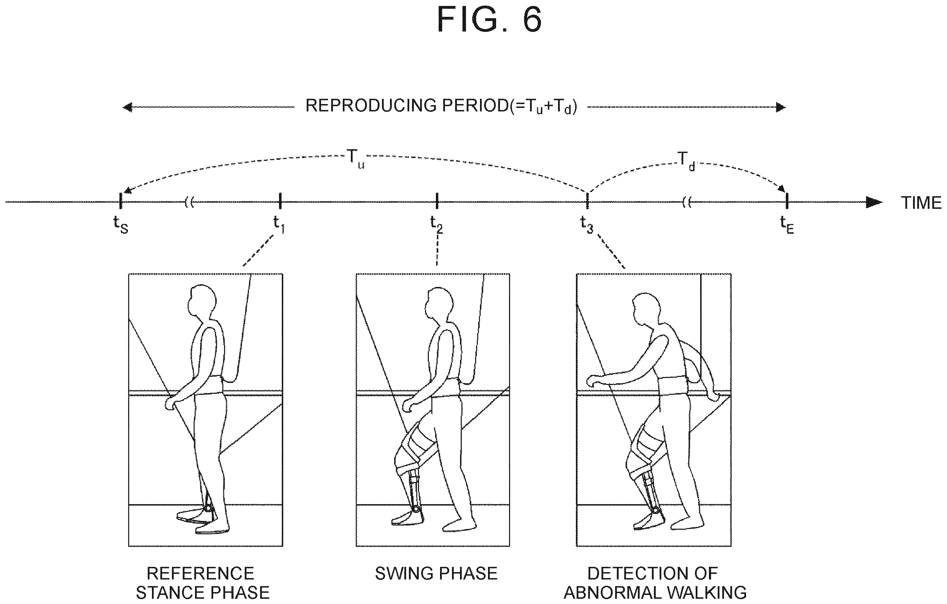

Next, a clipping operation for obtaining the video clip is described. The camera system 140 continuously captures images from start to end of the execution of the training, and the image processing unit 216 generates image data. The video clip is generated by clipping frame images associated with a reproducing period from a set start point to a set end point in the image data. FIG. 6 is an explanatory drawing of a reproducing period of an abnormal-walking video clip. A horizontal axis represents an elapse of time.

As in the illustration, it is assumed that the trainee 900 takes a posture of a reference stance phase at a time t₁, swings the leg at a time t₂, and stumbles at a time t₃. The reference stance phase is a phase immediately before the affected leg is swung. When the evaluation/determination unit 210a detects and determines that abnormal walking occurs based on a frame image captured at the time t₃, the overall control unit 210 reads a retrospective time Tᵤ with reference to the creation criterion table described later, and defines a start point of the video clip at a time t_{S} going back from the time t₃ by Tᵤ. In the creation criterion table, the retrospective time Tᵤ is set so that the time t_{S} goes back by some length from the time t₁ when the trainee 900 takes the posture of the reference stance phase.

The overall control unit 210 similarly reads a posterior time T_{d} with reference to the creation criterion table, and defines an end point of the video clip at a time t_{E} advancing from the time t₃ by T_{d}. In the creation criterion table, the posterior time T_{d} is set so that the time t_{E} at least advances by some length from a time when a next walking cycle may be started.

When a reproducing period (= Tᵤ + T_{d}) from the time t_{S} to the time t_{E} is determined, the image processing unit 216 generates a video clip by clipping frame images associated with this period. At this time, the image processing unit 216 also executes image processing to obtain an emphasis associated with a determination criterion serving as a basis for the determination that the abnormal walking occurs at the time t₃. In the image processing for obtaining the emphasis, a CG image may directly be embedded in each corresponding frame, or a CG layer to be superimposed on each frame may be created. To be exact, the timing of determination that the abnormal walking occurs differs from the timing of occurrence of the abnormal walking because a calculation time is required from the occurrence of the abnormal walking to the determination that the abnormal walking occurs. The calculation time is substantially negligible, and therefore the timing of the determination that the abnormal walking occurs may be regarded as being coincident with the timing of the occurrence of the abnormal walking. If the performance of the overall control unit 210 is low and the calculation time is not substantially negligible, the reproducing period may be adjusted in consideration of the calculation time so as to include a frame image at the timing of the occurrence of the abnormal walking.

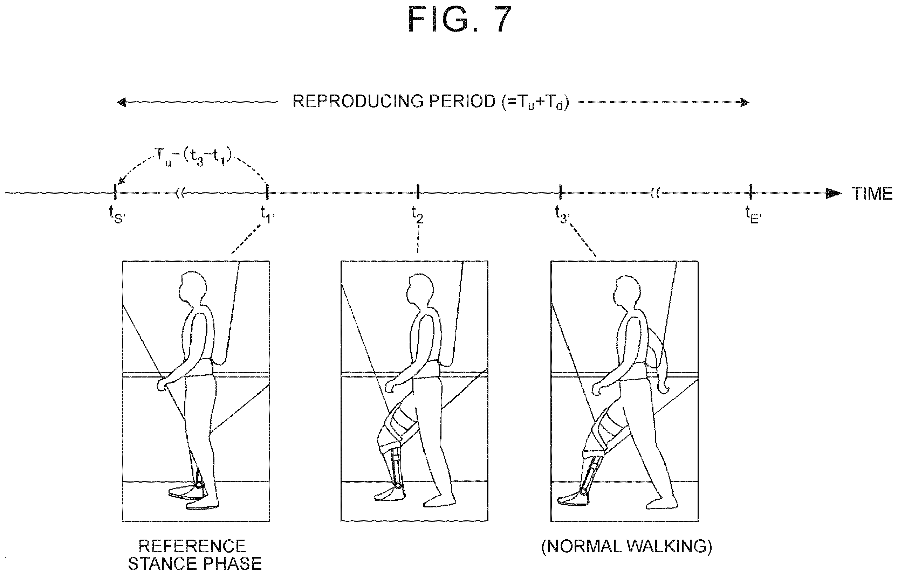

FIG. 7 is an explanatory drawing of a reproducing period of a normal-walking video clip. A horizontal axis represents an elapse of time similarly to FIG. 6. As described above, the normal-walking video clip is generated as the comparative video to the abnormal-walking video clip. Therefore, it is appropriate that the clipping operation for the normal-walking video clip be adjusted so that, when both video clips are reproduced in synchronization with each other, leg motions in both video clips are synchronized to some extent.

When the abnormal-walking video clip is generated as described with reference to FIG. 6, the image processing unit 216 searches for a predetermined continuous period in which no abnormal walking is detected. From the frame images in this period, the image processing unit 216 identifies a frame image showing a posture similar to that of the reference stance phase at the time t₁ in FIG. 6. Assuming that the time of the frame image is t_{1'}, the image processing unit 216 defines a start point of the normal-walking video clip at a time t_{S'} going back from the time t_{1'} by Tᵤ - (t₃ - t₁). Further, the image processing unit 216 defines an end point at a time t_{E'} advancing from the time t_{S'} by Tᵤ + T_{d}.

The image processing unit 216 defines the start point and the end point in this manner, and generates a normal-walking video clip by clipping frame images associated with the period. When simultaneous reproduction is started for the generated normal-walking video clip and the generated abnormal-walking video clip, the frame images of the reference stance phases are displayed at the same timing. In this state, a scene showing appropriate transition of the gait and a scene showing inappropriate transition of the gait are displayed by contrast. Since the reproducing periods of both video clips are Tᵤ + T_{d}, both video clips remain synchronized even if reproduced repeatedly.

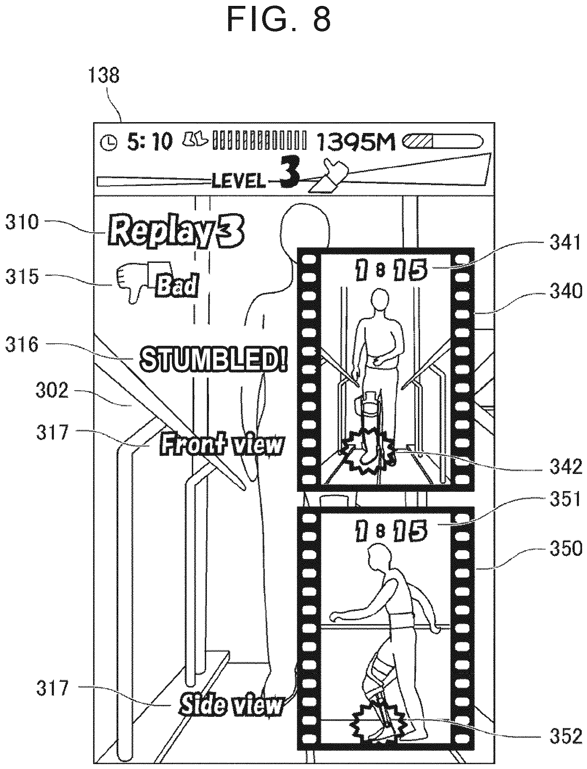

In the display example of the replay screen illustrated in FIG. 5, the abnormal-walking video clip is displayed adjacent to the normal-walking video clip associated with the abnormal-walking video clip, but the display example of the replay screen is not limited to the above display example. FIG. 8 illustrates a display example of another replay screen on the training monitor 138 after the execution of the training. The replay screen illustrated in FIG. 8 differs from the replay screen illustrated in FIG. 5 in that an abnormal-walking video clip 340 generated from a video captured by the front camera unit 141 adjoins an abnormal-walking video clip 350 generated from a video captured by the side camera unit 142. The video clips are generated by clipping frame images in the same period from the original videos. That is, the video clips capture scenes showing the trainee 900 in the same period in different directions.

An emphasis 342 is superimposed on the video clip 340 as a CG image to emphasize an abnormal part determined by the evaluation/determination unit 210a. The video clip 340 is obtained when the evaluation/determination unit 210a determines that the trainee 900 stumbles. Therefore, a CG image of an enclosure line that implies explosion is superimposed as the emphasis 342 so as to enclose a part of the affected leg of the trainee in contact with the ground. A time indication 341 is superimposed on the video clip 340 to indicate a time when the frame images are captured.

An emphasis 352 is superimposed on the video clip 350 as a CG image similarly to the emphasis 342 on the video clip 340. A time indication 351 is superimposed on the video clip 350 to indicate a time when the frame images are captured. As described above, the video clips 340 and 350 are generated by clipping frame images in the same period, and therefore the time indications 341 and 351 indicate the same time when simultaneous reproduction is started.

Direction indications 317 are superimposed near the video clips 340 and 350 in the camera image 302 to indicate imaging directions of the respective video clips. Since the video clip 340 is the video from the front camera unit 141, the corresponding direction indication 317 reads "Front View". Since the video clip 350 is the video from the side camera unit 142, the corresponding direction indication 317 reads "Side View".

The title 310, an attribute icon 315, and a determination comment 316 are superimposed on the camera image 302 similarly to the replay screen of FIG. 5. Since both the video clips 340 and 350 are the abnormal-walking video clips, one "Bad" icon is displayed as the attribute icon 315. The determination comment 316 is superimposed when the evaluation/determination unit 210a determines that the trainee 900 stumbles. Therefore, a corresponding comment "Stumbled!" is selected.

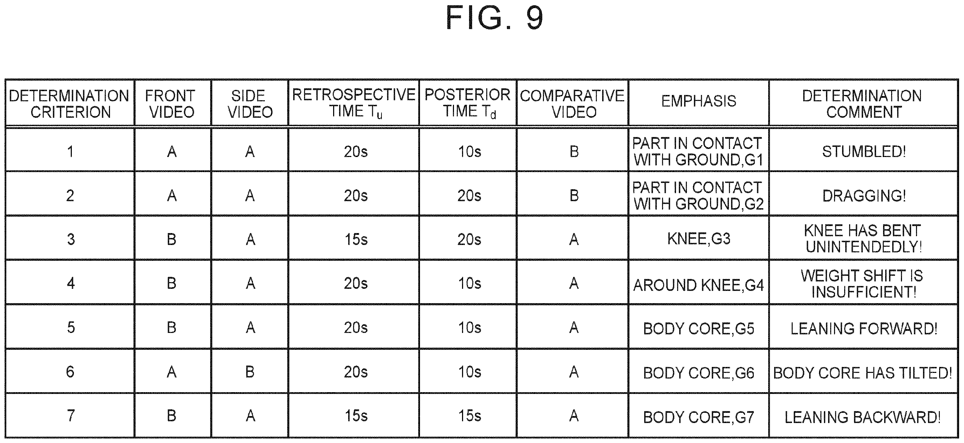

Next, the creation criterion table to be used for creating video clips is described. FIG. 9 is a diagram illustrating an example of the creation criterion table to be used for creating video clips. The creation criterion table defines how video clips are generated in conjunction with determination criteria. Specifically, the creation criterion table defines whether a video clip is generated by clipping a front video captured by the front camera unit 141 or a side video captured by the side camera unit 142 (in FIG. 9, "A" represents a case where the video clip is generated, and "B" represents a case where the video clip is not generated). The creation criterion table defines the retrospective time Tᵤ for determining a clipping start point, and the posterior time T_{d} for determining a clipping end point. The creation criterion table defines whether a normal-walking video clip is generated as a comparative video (in FIG. 9, "A" represents a case where the video clip is generated as a comparative video, and "B" represents a case where the video clip is not generated as a comparative video). The creation criterion table defines positions in a frame image and types of CG image related to the image processing for obtaining an emphasis on abnormal walking. The creation criterion table defines determination comments to be superimposed.

For example, the following seven criteria can be employed as the abnormal-walking determination criteria. The first criterion (determination criterion 1) is whether a distance from the hip joint to the ankle along a walking direction is equal to or larger than a reference value when the swing phase is ended and the affected leg is landed. When the distance is smaller than the reference value, determination is made that abnormal walking occurs because the affected leg cannot swing sufficiently. The scene in this case is prominently shown both in the front video and in the side video. Therefore, two video clips are generated by clipping the front video captured by the front camera unit 141 and the side video captured by the side camera unit 142. In this case, both video clips are placed as in the replay screen of FIG. 8. The retrospective time Tᵤ and the posterior time T_{d} are determined to be 20 seconds and 10 seconds, respectively, so that a sequence of scenes of the abnormal walking is included. The image processing for obtaining an emphasis is performed at a part of the affected leg in contact with the ground by using a G1 file containing a CG image prepared in the storage unit in advance. The determination comment is "Stumbled!" to indicate the abnormal walking shortly.

The second criterion (determination criterion 2) is whether a load on the sole is detected in the swing phase of the affected leg. When the load is detected, determination is made that abnormal walking occurs because of dragging. The scene in this case is prominently shown both in the front video and in the side video. Therefore, two video clips are generated by clipping the front video captured by the front camera unit 141 and the side video captured by the side camera unit 142. The image processing for obtaining an emphasis is performed at a part of the affected leg in contact with the ground by using a G2 file containing a CG image prepared in the storage unit in advance. The determination comment is "Dragging!" to indicate the abnormal walking shortly.

The third criterion (determination criterion 3) is whether the bending angle of the knee joint is equal to or larger than a reference angle in the stance phase of the affected leg. When the bending angle is smaller than the reference angle, determination is made that abnormal walking occurs because the knee joint lacks a force for supporting the upper body. The scene in this case is prominently shown in the side video, and therefore a video clip is generated by clipping the side video captured by the side camera unit 142. The degree of abnormality of this abnormal walking is well understandable in comparison with normal walking, and therefore a normal-walking video clip is generated as well. In this case, both video clips are placed as in the replay screen of FIG. 5. The image processing for obtaining an emphasis is performed at the position of the knee by using a G3 file containing a CG image prepared in the storage unit in advance. The determination comment is "Knee has bent unintendedly!" to indicate the abnormal walking shortly.

The fourth criterion (determination criterion 4) is whether the distance from the hip joint to the ankle along the walking direction is equal to or larger than a reference value when the stance phase is switched to the swing phase and the affected leg starts to swing. When the distance is smaller than the reference value, determination is made that abnormal walking occurs because the weight of the upper body cannot be shifted freely. The scene in this case is prominently shown in the side video, and therefore a video clip is generated by clipping the side video captured by the side camera unit 142. The degree of abnormality of this abnormal walking is well understandable in comparison with normal walking, and therefore a normal-walking video clip is generated as well. The image processing for obtaining an emphasis is performed at a position around the knee by using a G4 file containing a CG image prepared in the storage unit in advance. The determination comment is "Weight shift is insufficient!" to indicate the abnormal walking shortly.

The fifth criterion (determination criterion 5) is whether a forward tilt angle of the body core is equal to or larger than a reference angle in the stance phase of the affected leg. When the forward tilt angle is equal to or larger than the reference angle, determination is made that abnormal walking occurs because of a leaning-forward posture. The scene in this case is prominently shown in the side video, and therefore a video clip is generated by clipping the side video captured by the side camera unit 142. The degree of abnormality of this abnormal walking is well understandable in comparison with normal walking, and therefore a normal-walking video clip is generated as well. The image processing for obtaining an emphasis is performed at a body core position by using a G5 file containing a CG image prepared in the storage unit in advance. The determination comment is "Leaning forward!" to indicate the abnormal walking shortly.

The sixth criterion (determination criterion 6) is whether a tilt angle of the body core toward the affected leg is equal to or larger than a reference angle in the stance phase of the affected leg. When the tilt angle is equal to or larger than the reference angle, determination is made that abnormal walking occurs because of large deflection in the lateral direction. The scene in this case is prominently shown in the front video, and therefore a video clip is generated by clipping the front video captured by the front camera unit 141. The degree of abnormality of this abnormal walking is well understandable in comparison with normal walking, and therefore a normal-walking video clip is generated as well. The image processing for obtaining an emphasis is performed at the body core position by using a G6 file containing a CG image prepared in the storage unit in advance. The determination comment is "Body core has tilted!" to indicate the abnormal walking shortly.

The seventh criterion (determination criterion 7) is whether the forward tilt angle of the body core is equal to or larger than a reference angle in the swing phase of the affected leg. When the forward tilt angle is smaller than the reference angle, determination is made that abnormal walking occurs because the weight of the upper body cannot be shifted freely and the trainee is leaning backward. The scene in this case is prominently shown in the side video, and therefore a video clip is generated by clipping the side video captured by the side camera unit 142. The degree of abnormality of this abnormal walking is well understandable in comparison with normal walking, and therefore a normal-walking video clip is generated as well. The image processing for obtaining an emphasis is performed at the body core position by using a G7 file containing a CG image prepared in the storage unit in advance. The determination comment is "Leaning backward!" to indicate the abnormal walking shortly. In the determination criteria 2 to 7, the retrospective time Tᵤ and the posterior time T_{d} are determined to have given values in seconds, respectively, so that a sequence of scenes of the abnormal walking is included similarly to the determination criterion 1.

The reference value or the reference angle in each determination criterion may be changed depending on, for example, the age of the trainee 900 or the training level. The determination criterion is not limited to the determination criteria described above, but other determination criteria may be employed. For example, the determination criterion may be such that gripping of the handrail is recognized, or the harness tension unit 112 reels the harness wire 111. The evaluation/determination unit 210a determines whether any determination criterion is satisfied by using, for example, an analysis result from the image processing unit 216 or outputs from the sensors and drive signals from the drive units in the walking training apparatus 100 depending on the determination criterion. If the camera system 140 includes a camera unit other than the front camera unit 141 and the side camera unit 142, the creation criterion table may define whether a video clip is generated by using a video captured by this camera unit.

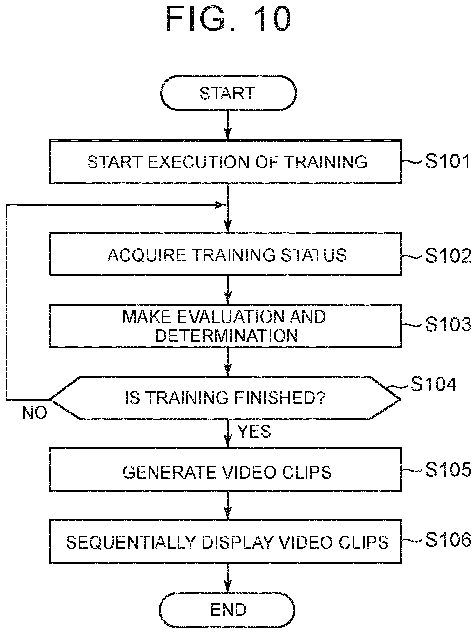

Next, description is given of control processing in a series of operations in the execution of the training. FIG. 10 is a diagram illustrating a processing flow in the series of operations in the execution of the training. In Step S101, the overall control unit 210 starts execution of training based on a training menu specified by the operator 910 or the trainee 900. Further, the overall control unit 210 starts imaging operations of the front camera unit 141 and the side camera unit 142.

When the execution of the training is started, the overall control unit 210 acquires a training status in Step S102. Specifically, the overall control unit 210 acquires images captured by the front camera unit 141 and the side camera unit 142, and causes the image processing unit 216 to perform image processing and also perform analysis processing for analyzing the gait. Further, the overall control unit 210 acquires output signals from the load sensor 222 and the like. The processing proceeds to Step S103, and the evaluation/determination unit 210a determines whether the walking action is normal or abnormal by applying the training status acquired in Step S 102 to the determination criteria. When the evaluation/determination unit 210a determines that the walking action is abnormal, the storage unit 217 stores the time of determination or frame image information associated with that time.

In Step S104, the overall control unit 210 determines whether the training is finished as planned. When the overall control unit 210 determines that the training is not finished, the processing returns to Step S102. When the overall control unit 210 determines that the training is finished, the processing proceeds to Step S105.

When the processing proceeds to Step S105, the image processing unit 216 reads, from the storage unit 217, the time of abnormality determination or the frame image information associated with that time, which is stored in Step S103. Then, video clips are generated, as described above, by clipping videos in a period including the time of abnormality determination from the videos captured by the front camera unit 141 and the side camera unit 142 with reference to the creation criterion table. At this time, the video clips are subjected to image processing to emphasize an abnormal part of the abnormal walking determined in Step S103.

The processing proceeds to Step S106, and the overall control unit 210 causes, via the display control unit 213, the training monitor 138 to sequentially display the video clips subjected to the image processing as illustrated in FIG. 5 or FIG. 8. When the overall control unit 210 finishes displaying all the generated video clips, the overall control unit 210 terminates the series of processing steps.

In the embodiment described above, description is given of the example in which the video clips are displayed immediately after the execution of the training. The overall control unit 210 may store the video clips in the storage unit 217, and display the video clips prior to next training to be executed by the same trainee. Even if the video clips are displayed in this manner, the trainee can quickly and intuitively review the previous execution of the training prior to a new execution of the training.

In the embodiment described above, the image processing unit 216 determines the clipping period for a video clip with reference to the times defined in the creation criterion table in regard to a determination criterion determined to be applicable by the evaluation/determination unit 210a. The clipping period is not limited to the clipping period described above. For example, the clipping period may be determined based on a walking cycle of the trainee. For example, the evaluation/determination unit 210a can detect the walking cycle of the trainee based on repetition of the posture of the reference stance phase. The image processing unit 216 determines a clipping period for a video clip based on the walking cycle detected by the evaluation/determination unit 210a and defined cycles in the creation criterion table, such as three retrospective cycles and two posterior cycles. When the video clip is generated in this manner, for example, a trainee whose leg motion speed is low can recognize the entire scene of the abnormal walking more securely.

In the embodiment described above, the image processing unit 216 performs the processing of superimposing a CG image on a frame image as the image processing for emphasizing an abnormal part. The image processing for emphasizing an abnormal part is not limited to this processing. For example, the abnormal part may be trimmed and enlarged, or the outline of the abnormal part may be emphasized by a red line. The camera system 140 may include not only the camera units configured to capture the entire body of the trainee 900, but also a camera unit configured to capture a specific part of the trainee 900, such as feet. The image processing unit 216 may generate a video clip by clipping a video captured by the camera unit configured to capture a specific part.

The emphasizing processing may involve not only displaying an abnormal part, but also displaying suggestion to resolve abnormal walking. For example, when an abnormality of swing difficulty is detected, the toe that is insufficiently lifted off the ground is enclosed by a red circle, and a blue upward arrow is displayed near the ankle to prompt the trainee to imagine the lifting. When the suggestion to resolve the abnormal walking is displayed in a different color, the trainee can intuitively recognize how to resolve the abnormal walking.

In the embodiment described above, description is given of the configuration in which the walking training apparatus 100 includes the image processing unit 216, the storage unit 217, and the evaluation/determination unit 210a, but those functions may be provided in a server connected to the walking training apparatus via a network. In this case, the server that executes a control program for controlling the walking training apparatus acquires necessary information from the walking training apparatus, determines whether abnormal walking occurs, generates a video clip, emphasizes an abnormal part, and displays the video clip on the monitor of the walking training apparatus.

In the walking training system, all the functional elements are not necessarily integrated into the walking training apparatus 100. For example, the function of the evaluation/determination unit 210a may be provided in a calculation unit of a server connected to the walking training apparatus 100 via a network. In this case, the server transmits an abnormal-walking determination result to the walking training apparatus 100. The overall control unit 210 of the walking training apparatus 100 achieves display similar to that of the embodiment described above by using the transmitted determination result. Thus, the walking training system may include the server and the walking training apparatus 100.

## Claims

1. A walking training system (100) comprising:
a treadmill (131) configured to prompt a trainee (900) to walk;
a camera system configured to image the trainee (900) at an angle of view at which at least a part of a gait of the trainee (900) walking on the treadmill (131) is recognizable;
a determination unit (210a) configured to determine whether abnormal walking occurs in the trainee (900) based on a plurality of predetermined determination criteria;
an image processing unit (216) configured to:
clip, as a video clip, a video in a predetermined period including a timing when the determination unit (210a) determines that the abnormal walking occurs from a video captured by the camera system; and
perform image processing to emphasize an abnormal part of the abnormal walking determined by the determination unit (210a); and
a display control unit (213) configured to cause a display unit to display the video clip subjected to the image processing.

2. The walking training system (100) according to claim 1, wherein:
the camera system includes a plurality of cameras (141, 142) configured to image the trainee (900) in different directions; and
the image processing unit (216) is configured to determine which of videos captured by the cameras (141, 142) is clipped as the video clip based on a determination criterion determined to be applicable by the determination unit (210a) out of the determination criteria.

3. The walking training system (100) according to claim 2, wherein:
the image processing unit (216) is configured such that, when the determination criterion determined to be applicable by the determination unit (210a) is a specific determination criterion, the videos captured by the cameras (141, 142) are clipped as the video clips; and
the display control unit (213) is configured to cause the display unit to display the video clips in synchronization with each other.

4. The walking training system (100) according to any one of claims 1 to 3, wherein:
the image processing unit (216) is configured to clip, as a comparative video, a video in a normal-walking period in which the determination unit (210a) does not determine that the abnormal walking occurs; and
the display control unit (213) is configured to cause the display unit to display the comparative video such that the comparative video adjoins the video clip.

5. The walking training system (100) according to any one of claims 1 to 4, wherein the image processing unit (216) is configured to determine a clipping period for the video clip based on the determination criterion determined to be applicable by the determination unit (210a).

6. The walking training system (100) according to any one of claims 1 to 5, wherein the image processing unit (216) is configured to determine the clipping period for the video clip based on a walking cycle of the trainee (900).

7. A non-transitory storage medium storing a control program for a walking training system (100) including a treadmill (131) configured to prompt a trainee (900) to walk, and a camera system configured to image the trainee (900) at an angle of view at which at least a part of a gait of the trainee (900) walking on the treadmill (131) is recognizable, the non-transitory storage medium storing instructions that are executable by one or more processors and that cause the one or more processors to perform functions comprising:
determining whether abnormal walking occurs in the trainee (900) based on a plurality of predetermined determination criteria;
clipping, as a video clip, a video in a predetermined period including a timing when determination is made that the abnormal walking occurs from a video captured by the camera system;
performing image processing on the video clip to emphasize an abnormal part of the determined abnormal walking; and
causing a display unit to display the video clip subjected to the image processing.

8. A control method for a walking training system (100) including a treadmill (131) configured to prompt a trainee (900) to walk, and a camera system configured to image the trainee (900) at an angle of view at which at least a part of a gait of the trainee (900) walking on the treadmill (131) is recognizable, the control method comprising:
determining whether abnormal walking occurs in the trainee (900) based on a plurality of predetermined determination criteria;
clipping, as a video clip, a video in a predetermined period including a timing when determination is made that the abnormal walking occurs from a video captured by the camera system;
performing image processing on the video clip to emphasize an abnormal part of the determined abnormal walking; and
causing a display unit to display the video clip subjected to the image processing.

## Patentansprüche

1. Gehtrainingssystem (100), umfassend:
ein Laufband (131), das dafür ausgelegt ist, einen Trainierenden (900) zum Gehen aufzufordern;
ein Kamerasystem, das dafür ausgelegt ist, den Trainierenden (900) in einem Sichtwinkel abzubilden, in dem wenigstens ein Teil eines Gangs des Trainierenden (900), der auf dem Laufband (131) geht, erkennbar ist;
eine Bestimmungseinheit (210a), die dafür ausgelegt ist zu bestimmen, ob ein abnormaler Gang bei dem Trainierenden (900) vorliegt, basierend auf einer Mehrzahl von vorbestimmten Bestimmungskriterien;
eine Bildverarbeitungseinheit, die ausgelegt ist zum:
Ausschneiden, als Videoclip, eines Videos in einem vorbestimmten Zeitraum, welcher einen Zeitpunkt beinhaltet, an dem die Bestimmungseinheit (210a) anhand eines Videos, das von dem Kamerasystem erfasst wird, bestimmt, dass der abnormale Gang vorliegt; und
Durchführen einer Bildverarbeitung, um einen abnormalen Teil des abnormalen Gangs, der durch die Bestimmungseinheit (210a) bestimmt wird, hervorzuheben; und
eine Anzeigesteuereinheit (213), die dafür ausgelegt ist, eine Anzeigeeinheit zu veranlassen, den Videoclip, welcher der Bildverarbeitung unterzogen wurde, anzuzeigen.

2. Gehtrainingssystem (100) gemäß Anspruch 1, wobei:
das Kamerasystem mehrere Kameras (141, 142) beinhaltet, die dafür ausgelegt sind, den Trainierenden (900) in unterschiedlichen Richtungen abzubilden; und
die Bildverarbeitungseinheit (216) dafür ausgelegt ist zu bestimmen, welches der von den Kameras (141, 142) erfassten Videos als Videoclip ausgeschnitten wird, basierend auf einem Bestimmungskriterium, das von der Bestimmungseinheit (210a) als anzuwendendes Kriterium aus den Bestimmungskriterien bestimmt wird.

3. Gehtrainingssystem (100) gemäß Anspruch 2, wobei:
die Bildverarbeitungseinheit (216) dergestalt ausgelegt ist, dass, wenn das von der Bestimmungseinheit (210a) als anzuwendendes Kriterium bestimmte Bestimmungskriterium ein spezifisches Bestimmungskriterium ist, die von den Kameras (141, 142) erfassten Videos als die Videoclips ausgeschnitten werden; und
die Anzeigesteuereinheit (213) dafür ausgelegt ist, die Anzeigeeinheit zu veranlassen, die Videoclips in Synchronisation miteinander anzuzeigen.

4. Gehtrainingssystem (100) gemäß einem der Ansprüche 1 bis 3, wobei:
die Bildverarbeitungseinheit (216) dafür ausgelegt ist, als Vergleichsvideo ein Video in einem Normalgehzeitraum auszuschneiden, in dem die Bestimmungseinheit (210a) nicht bestimmt, dass der abnormale Gang vorliegt; und
die Anzeigesteuereinheit (213) dafür ausgelegt ist, die Anzeigeeinheit zu veranlassen, das Vergleichsvideo so anzuzeigen, dass das Vergleichsvideo an den Videoclip angrenzt.

5. Gehtrainingssystem (100) gemäß einem der Ansprüche 1 bis 4, wobei die Bildverarbeitungseinheit (216) dafür ausgelegt ist, einen Ausschnittzeitraum für den Videoclip zu bestimmen, basierend auf dem Bestimmungskriterium, das von der Bestimmungseinheit (210a) als anzuwendendes Kriterium bestimmt wird.

6. Gehtrainingssystem (100) gemäß einem der Ansprüche 1 bis 5, wobei die Bildverarbeitungseinheit (216) dafür ausgelegt ist, den Ausschnittzeitraum für den Videoclip basierend auf einem Gehzyklus des Trainierenden (900) zu bestimmen.

7. Nicht-transitorisches Datenspeichermedium, das ein Steuerprogramm für ein Gehtrainingssystem (100) speichert, beinhaltend ein Laufband (131), das dafür ausgelegt ist, einen Trainierenden (900) zum Gehen aufzufordern, und ein Kamerasystem, das dafür ausgelegt ist, den Trainierenden (900) in einem Sichtwinkel abzubilden, in dem wenigstens ein Teil eines Gangs des Trainierenden (900), der auf dem Laufband (131) geht, erkennbar ist, wobei das nicht-transitorische Datenspeichermedium Anweisungen speichert, die von einem oder mehreren Prozessoren ausgeführt werden können und die die ein oder mehreren Prozessoren veranlassen, Funktionen durchzuführen, welche umfassen:
Bestimmen, ob ein abnormaler Gang bei dem Trainierenden (900) vorliegt, basierend auf einer Mehrzahl von vorbestimmten Bestimmungskriterien;
Ausschneiden, als Videoclip, eines Videos in einem vorbestimmten Zeitraum, welcher einen Zeitpunkt beinhaltet, an dem anhand eines Videos, das von dem Kamerasystem erfasst wird, eine Bestimmung erfolgt, dass der abnormale Gang vorliegt;
Durchführen einer Bildverarbeitung an dem Videoclip, um einen abnormalen Teil des bestimmten abnormalen Gangs hervorzuheben; und
Veranlassen einer Anzeigeeinheit, den Videoclip, welcher der Bildverarbeitung unterzogen wurde, anzuzeigen.

8. Steuerungsverfahren für ein Gehtrainingssystem (100) mit einem Laufband (131), das dafür ausgelegt ist, einen Trainierenden (900) zum Gehen aufzufordern, und einem Kamerasystem, das dafür ausgelegt ist, den Trainierenden (900) in einem Sichtwinkel abzubilden, in dem wenigstens ein Teil eines Gangs des Trainierenden (900), der auf dem Laufband (131) geht, erkennbar ist, wobei das Steuerungsverfahren umfasst:
Bestimmen, ob ein abnormaler Gang bei dem Trainierenden (900) vorliegt, basierend auf einer Mehrzahl von vorbestimmten Bestimmungskriterien;
Ausschneiden, als Videoclip, eines Videos in einem vorbestimmten Zeitraum, welcher einen Zeitpunkt beinhaltet, an dem anhand eines Videos, das von dem Kamerasystem erfasst wird, eine Bestimmung erfolgt, dass der abnormale Gang vorliegt;
Durchführen einer Bildverarbeitung an dem Videoclip, um einen abnormalen Teil des bestimmten abnormalen Gangs hervorzuheben; und
Veranlassen einer Anzeigeeinheit, den Videoclip, welcher der Bildverarbeitung unterzogen wurde, anzuzeigen.

## Revendications

1. Système d'entraînement à la marche (100) comprenant :
un tapis roulant (131) configuré pour inviter la personne s'entraînant (900) à marcher ;
un système de caméra configuré pour prendre des images de la personne s'entraînant (900) à un angle de vue auquel au moins une partie de l'allure de la personne s'entraînant (900) marchant sur le tapis roulant (131) est reconnaissable ;
une unité de détermination (210a) configurée pour déterminer si une marche anormale se produit chez la personne s'entraînant (900) sur la base d'une pluralité de critères de détermination prédéterminés ;
une unité de traitement d'images (216) configurée pour :
couper, en tant que clip vidéo, une vidéo dans une période prédéterminée comprenant un moment où l'unité de détermination (210a) détermine que la marche anormale se produit à partir d'une vidéo capturée par le système de caméra ; et
effectuer un traitement d'images pour mettre en évidence une partie anormale de la marche anormale déterminée par l'unité de détermination (210a) ; et
une unité de commande d'affichage (213) configurée pour amener une unité d'affichage à afficher le clip vidéo soumis au traitement d'images.

2. Système d'entraînement à la marche (100) selon la revendication 1, dans lequel :
le système de caméra comprend une pluralité de caméras (141, 142) configurées pour prendre des images de la personne s'entraînant (900) dans différentes directions ; et
l'unité de traitement d'images (216) est configurée pour déterminer laquelle des vidéos capturées par les caméras (141, 142) est coupée en tant que clip vidéo sur la base d'un critère de détermination déterminé comme étant applicable par l'unité de détermination (210a) parmi les critères de détermination.

3. Système d'entraînement à la marche (100) selon la revendication 2, dans lequel :
l'unité de traitement d'images (216) est configurée de telle sorte que, lorsque le critère de détermination déterminé comme étant applicable par l'unité de détermination (210a) est un critère de détermination spécifique, les vidéos capturées par les caméras (141, 142) sont coupées en tant que clips vidéo ; et
l'unité de commande d'affichage (213) est configurée pour que l'unité d'affichage affiche les clips vidéo de manière synchronisée les uns avec les autres.

4. Système d'entraînement à la marche (100) selon l'une quelconque des revendications 1 à 3, dans lequel :
l'unité de traitement d'images (216) est configurée pour couper, en tant que vidéo comparative, une vidéo dans une période de marche normale dans laquelle l'unité de détermination (210a) ne détermine pas que la marche anormale se produit ; et
l'unité de commande d'affichage (213) est configurée pour amener l'unité d'affichage à afficher la vidéo comparative de manière à ce que la vidéo comparative soit adjacente au clip vidéo.

5. Système d'entraînement à la marche (100) selon l'une quelconque des revendications 1 à 4, dans lequel l'unité de traitement d'images (216) est configurée pour déterminer une période de découpage pour le clip vidéo sur la base du critère de détermination déterminé comme étant applicable par l'unité de détermination (210a).

6. Système d'entraînement à la marche (100) selon l'une quelconque des revendications 1 à 5, dans lequel l'unité de traitement d'images (216) est configurée pour déterminer la période de découpage pour le clip vidéo sur la base d'un cycle de marche de la personne s'entraînant (900) .

7. Support de stockage non transitoire stockant un programme de commande pour un système d'entraînement à la marche (100) comprenant un tapis roulant (131) configuré pour inviter une personne s'entraînant (900) à marcher, et un système de caméra configuré pour prendre des images de la personne s'entraînant (900) à un angle de vue auquel au moins une partie de l'allure de la personne s'entraînant (900) marchant sur le tapis roulant (131) est reconnaissable, le support de stockage non transitoire stockant des instructions qui sont exécutables par un ou plusieurs processeurs et qui amènent les un ou plusieurs processeurs à exécuter des fonctions comprenant les étapes suivantes :
déterminer si une marche anormale se produit chez la personne s'entraînant (900) sur la base d'une pluralité de critères de détermination prédéterminés ;
couper, en tant que clip vidéo, une vidéo dans une période prédéterminée comprenant un moment où il est déterminé que la marche anormale se produit à partir d'une vidéo capturée par le système de caméra ;
effectuer un traitement d'images sur le clip vidéo pour mettre en évidence une partie anormale de la marche anormale déterminée ; et
amener une unité d'affichage à afficher le clip vidéo soumis au traitement d'images.

8. Procédé de commande pour un système d'entraînement à la marche (100) comprenant un tapis roulant (131) configuré pour inviter une personne s'entraînant (900) à marcher, et un système de caméra configuré pour prendre des images de la personne s'entraînant (900) à un angle de vue auquel au moins une partie de l'allure de la personne s'entraînant (900) marchant sur le tapis roulant (131) est reconnaissable, le procédé de commande comprenant les étapes suivantes :
déterminer si une marche anormale se produit chez la personne s'entraînant (900) sur la base d'une pluralité de critères de détermination prédéterminés ;
couper, en tant que clip vidéo, une vidéo dans une période prédéterminée comprenant un moment où il est déterminé que la marche anormale se produit à partir d'une vidéo capturée par le système de caméra ;
effectuer un traitement d'images sur le clip vidéo pour mettre en évidence une partie anormale de la marche anormale déterminée ; et
amener une unité d'affichage à afficher le clip vidéo soumis au traitement d'images.
